# EUROPEAN PATENT APPLICATION

(11) **EP 3 760 111 A1**
(43) Date of publication of application: **06.01.2021**
(21) Application number: 18910947.3
(22) Date of filing: 07.12.2018
(51) Int. Cl.: A61B 5/021

(54) **EXPANDABLE MULTIPLE PHYSIOLOGICAL PARAMETER MONITORING RING**

(30) Priority: 21.03.2018 CN 201810233577
(71) Applicant: Hangzhou Megasens Technologies Co., Ltd., Hangzhou, Zhejiang 310052 (CN)
(72) Inventor: ZHOU, Congcong, Zhejiang 310052 (CN); HU, Jun, Zhejiang 310052 (CN); YUAN, Haiquan, Zhejiang 310052 (CN)
(74) Representative: FARAGO Patentanwälte
(86) International application number: PCT/CN2018/119853
(87) International publication number: WO 2019/179175

(57) **Abstract**

An expandable multi-physiological parameter monitoring ring, comprising a microprocessor module (1) and an internal sensor module (3) connected to the microprocessor module (1), and further comprising an external module interface (4) detachably connected to the microprocessor module (1) and used for connecting an external sensor module (5). The microprocessor module (1) is configured to: when the external module interface (4) is connected to the external sensor module (5), obtain measurement signals from the external sensor module (5) and the internal sensor module (3), and process the measurement signals to obtain a difference between a measurement signal of the external sensor module (5) and a measurement signal of the internal sensor module (3); and when the external module interface (4) is not connected to the external sensor module (5), calibrate, according to the difference, a physiological parameter obtained by the internal sensor module (3). The monitoring ring can improve accuracy of physiological parameter measurement at a fingertip, and can implement low load, portability, and comfort.

## Description

### Technical Field

The present invention relates to the field of health medical device, and specifically, to an expandable multi-physiological parameter monitoring ring.

### Background of Art

The traditional measurement device for monitoring physiological parameters, such as electrocardiogram and blood pressure etc., is mainly a multi-physiological parameter monitor, which usually requires a variety of lead wires and connections of accessories such as cables and cuffs, and is used under the guidance of professional technicians, which is inapplicable to daily monitoring. The existing small-sized, low-load multi-physiological parameter monitoring device mainly uses a pulse wave transit time method when monitoring a blood pressure, and the principle is to establish a mathematical model between a pulse wave transit time (PTT) or a pulse wave propagation velocity (PWV) and an arterial blood pressure, and then the blood pressure is calculated by measuring pulse wave parameters. A time difference between an electrocardiographic (ECG) signal and a photoplethysmographic (PPG) signal is usually used to obtain the pulse wave transit time. The pulse wave is affected by many factors in the process of propagating along an artery vessel. Therefore, to ensure the reliability of the mathematical model (that is, the correlation between the pulse wave transit time and the arterial blood pressure), the photoplethysmographic (PPG) signal needs to be measured near the main artery, such as the brachial artery, the radial artery, or the ulnar artery. However, in order to implement portability and comfort, the existing small-sized and low-load multi-physiological parameter monitoring device generally cannot meet the above requirements. Consequently, there is a problem that the blood pressure measurement accuracy is not high.

### Summary of the invention

To solve all or some of the above-mentioned problems, the present invention provides an expandable multi-physiological parameter monitoring ring.

An expandable multi-physiological parameter monitoring ring is disclosed in an implementation of the present invention, including a microprocessor module and an internal sensor module connected to the microprocessor module, wherein further including an external module interface used for connecting an external sensor module. The external module interface is detachably connected to the microprocessor module.

The microprocessor module is configured to: when the external module interface is connected to the external sensor module, obtain measurement signals from the external sensor module and the internal sensor module, and process the measurement signals to obtain a difference between a measurement signal of the external sensor module and a measurement signal of the internal sensor module; and
when the external module interface is not connected to the external sensor module, calibrate, according to the difference, a physiological parameter obtained by the external sensor module.

In an example, the physiological parameter is a pulse wave transmit time or a pulse wave propagation velocity.

In an example, the microprocessor module is configured to obtain a difference value between the physiological parameter calculated based on the measurement signal of the external sensor module and the physiological parameter calculated based on the measurement signal of the internal sensor module as the difference.

In an example, the internal sensor module includes an internal pulse wave measurement module and an internal electrocardiogram monitoring module.

In an example, the internal pulse wave measurement module includes an internal photoplethysmography analog front end and an internal photoplethysmography module. When the ring is worn, the internal photoplethysmography module is in contact with a finger, and the internal photoplethysmography analog front end processes a signal from the internal photoplethysmography module.

In an example, the internal photoplethysmography module includes a photodiode protrudingly disposed on an inner circumferential surface of the ring and at least one light emitting diode array that is central symmetrical with respect to the photodiode. An elastic device is disposed at the bottom of the photodiode. When the ring is worn, the elastic device is pressed, so that the photodiode and the at least one light emitting diode array are in close contact with the finger, and light emitted by the light emitting diode array is attenuated by the finger and then received by the photodiode.

In an example, the internal electrocardiogram monitoring module includes an internal electrocardiogram monitoring analog front end and at least two surface electrode interfaces. The surface electrode interface is used for mounting an electrode. When the finger ring is worn, the electrode is in contact with a finger, and the internal electrocardiogram monitoring analog front end processes a signal from the surface electrode interface.

In an example, the ring further includes a function expansion module connected to the microprocessor module. The internal sensor module and the external module interface are connected to the microprocessor module by the function expansion module. The function expansion module is configured to receive a control signal of the microprocessor module, so as to enable one or more of the external module interface and respective submodules of the internal sensor module to work.

Comparing the embodiments of the present invention with the prior art, the main differences and effects are as follows: When corresponding physiological parameters cannot be accurately calculated according to physiological signals collected at a fingertip, an expandable multi-physiological parameter monitoring ring is allowed to obtain measurement signals from an internal sensor module and an external sensor module, and calibrate, according to a difference between internal measurement and external measurement, a physiological parameter obtained by using only the internal sensor module at a subsequent moment. In this way, accuracy of physiological parameter measurement at the fingertip is improved.

Further, the external sensor module may be connected only when measurement is initially performed, connection to the external sensor module can be removed after the measurement signal is obtained, and even the external module interface can be removed. The expandable multi-physiological parameter monitoring ring in the embodiments of the present invention can still implement low load, portability, and comfort.

Further, the central symmetrical structure of the internal photoplethysmography module and the elastic device at the bottom can effectively ensure pulse wave signal quality and improve accuracy of physiological parameter measurement.

### BRIEF DESCRIPTION OF THE DRAWINGS

FIG. 1 is a schematic diagram of function modules of an expandable multi-physiological parameter monitoring ring according to an embodiment of the present invention;
FIG. 2 is a schematic diagram of function modules of an expandable multi-physiological parameter monitoring ring that can implement blood pressure monitoring according to an embodiment of the present invention;
FIG. 3 is a schematic structural diagram of an expandable multi-physiological parameter monitoring ring that can implement blood pressure monitoring according to an embodiment of the present invention;
FIG. 4 is a top view of an internal photoplethysmography module according to an embodiment of the present invention;
FIG. 5 is a front view of an internal photoplethysmography module according to an embodiment of the present invention; and
FIG. 6 is a schematic connection diagram of an external sensor module according to an embodiment of the present invention.

### DESCRIPTION OF THE EMBODIMENTS

In the following description, numerous technical details are provided for a reader to better understand the present application. However, persons of ordinary skill in the art can understand that the technical solutions that the claims of the present application request to protect can be implemented without these technical details and various changes and modifications based on the following embodiments.

To make the objectives, technical solutions, and advantages of the embodiments of the present invention clearer, the following describes the embodiments of the present invention in detail with reference to the accompanying drawings.

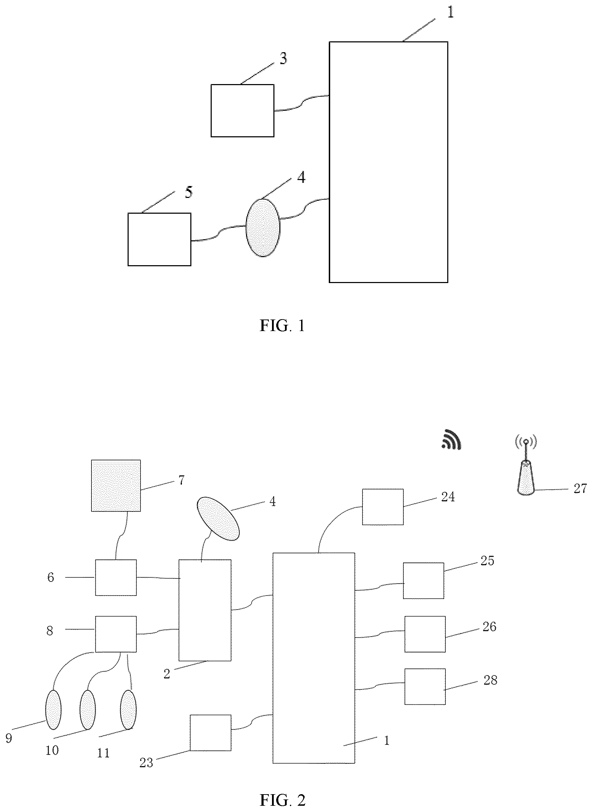

FIG. 1 is a schematic diagram of function modules of an expandable multi-physiological parameter monitoring ring according to an embodiment of the present invention. As shown in FIG. 1, the expandable multi-physiological parameter monitoring ring includes a microprocessor module 1 and an internal sensor module 3 connected to the microprocessor module 1, and further includes an external module interface 4 used for connecting an external sensor module 5. The external module interface 4 is detachably connected to the microprocessor module 1. The microprocessor module 1 is configured to: when the external module interface 4 is connected to the external sensor module 5, obtain measurement signals from the external sensor module 5 and the internal sensor module 3, and process the measurement signals to obtain a difference between a measurement signal of the external sensor module 5 and a measurement signal of the internal sensor module 3, and when the external module interface 4 is not connected to the external sensor module 5, calibrate, according to the difference, a physiological parameter obtained by the internal sensor module 3.

When corresponding physiological parameters cannot be accurately calculated according to the physiological signals collected at a fingertip, the expandable multi-physiological parameter monitoring ring is allowed to obtain measurement signals from the internal sensor module and the external sensor module, and process the measurement signals to obtain a difference between a measurement signal of the external sensor module and a measurement signal of the internal sensor module, and a physiological parameter obtained by using only the internal sensor module at a subsequent moment may be calibrated according to a difference. In this way, accuracy of physiological parameter measurement at the fingertip is improved. In addition, the external sensor module may be connected only when measurement is initially performed, connection to the external sensor module can be removed after the measurement signal is obtained, and even the external module interface can be removed. The expandable multi-physiological parameter monitoring ring in the embodiments of the present invention can still implement low load, portability, and comfort.

In this embodiment of the present invention, the physiological parameter may be any physiological parameter that is measured at a fingertip and that needs to be calibrated. In the following description, blood pressure monitoring based on a pulse wave transmit time method is used as an example to describe the structure and the function modules of the expandable multi-physiological parameter monitoring ring. In this case, the physiological parameter is pulse wave transmit time or pulse wave propagation velocity.

A schematic diagram of function modules of an expandable multi-physiological parameter monitoring ring for blood pressure monitoring is shown in FIG. 2. In this embodiment of the present invention, a microprocessor module 1 may be directly connected to an internal sensor module 3 and an external module interface 4, or may be connected to the internal sensor module 3 and the external module interface 4 by using an expansion control module 2. The expansion control module 2 may be a set of analog switches. Under the control of the microprocessor module 1, one or more of the external module interface 4 and respective submodules of the internal sensor module 3 are selected to work. The blood pressure monitoring based on the pulse wave transmit time method needs to simultaneously measure an electrocardiogram (ECG) signal and a photoplethysmography (PPG) signal. Correspondingly, the internal sensor module 3 includes an internal electrocardiogram monitoring module and an internal pulse wave measurement module. The internal pulse wave measurement module implements measurement of the photoplethysmography (PPG) signal, and may include an internal photoplethysmography analog front end 6 and an internal photoplethysmography module 7. When the ring is worn, the internal photoplethysmography module 7 is in contact with a finger, and the internal photoplethysmography analog front end 6 processes (for example, amplifies or filters) a signal from the internal photoplethysmography module 7, and is optionally connected to the expansion control module 2. The internal electrocardiogram module implements measurement of the electrocardiogram (ECG) signal, and may include an internal electrocardiogram analog front end 8 and at least two surface electrode interfaces. The surface electrode interface is used for mounting an electrocardiogram monitoring electrode. When the ring is worn, the electrode is in contact with a finger, and the internal electrocardiogram analog front end 8 processes (for example, amplifies or filters) a signal from the surface electrode interface, and is optionally connected to the expansion control module 2. Preferably, the internal electrocardiogram monitoring module includes three surface electrode interfaces, for example, three electrodes 9, 10, and 11 as shown in FIG. 2.

The microprocessor module 1 in this embodiment of the present invention may be but not limited to a minimum system module, for example, 2540 of Ti, or nRF52832, that supports embedded system development and that is developed by Ti Company or Nordic Company.

In this embodiment of the present invention, the internal photoplethysmography analog front end 6 may be an integrated analog front end, such as ADPD174 of ADI, or may be an analog circuit built by a discrete resistor-capacitor and an operational amplifier. The internal electrocardiogram monitoring analog front end 8 may be an integrated analog front end, such as AD8232 of ADI, or may be an analog circuit built by a discrete resistor-capacitor, an instrumentation amplifier, and an operational amplifier.

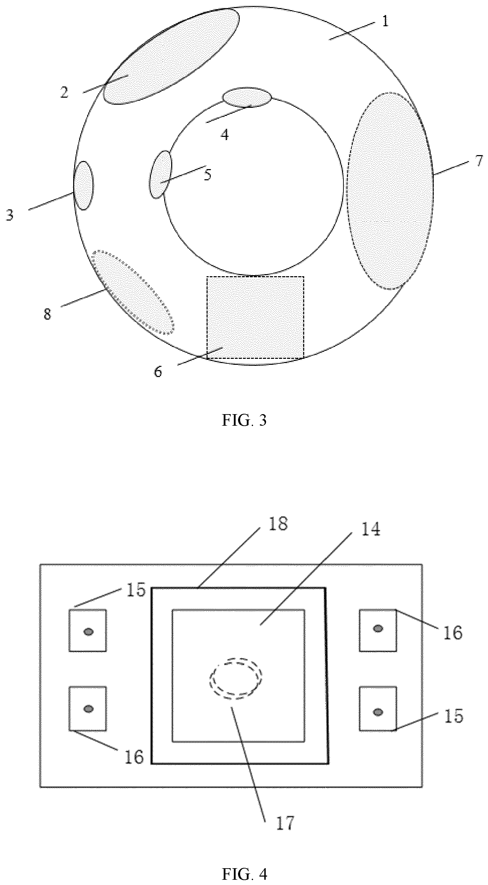

FIG. 3 is a schematic structural diagram of an expandable multi-physiological parameter monitoring ring for blood pressure monitoring corresponding to the ring in FIG. 2. 12 indicates an inner circumferential surface of the expandable multi-physiological parameter monitoring ring, 13 indicates an outer circumferential surface of the expandable multi-physiological parameter monitoring ring, and a sensor and a circuit system inside the ring are designed based on a flexible circuit. Three electrode interfaces 9, 10, and 11 are shown in FIG. 3. The electrode interface 9 is located on the outer circumferential surface 13 of the ring, and the electrode interfaces 10 and 11 are located on the inner circumferential surface 12 of the ring. When the ring is worn, electrodes corresponding to the electrode interfaces 10 and 11 are in contact with a finger, and an electrode corresponding to the electrode interface 9 is in contact with the other hand on which the ring is not worn, which constitutes a three-electrode-lead system. Optionally, the electrode interfaces 9 and 10 are located on the outer circumferential surface 13 of the ring, and the electrode interface 11 is located on the inner circumferential surface 12 of the ring. When the ring is worn, an electrode corresponding to the electrode interface 11 is in contact with a finger, and electrodes corresponding to the electrode interfaces 9 and 10 are in contact with the other hand on which a ring is not worn, which constitutes a three-electrode-lead system. Preferably, a dry electrode or a fabric electrode is selected as an electrocardiogram monitoring electrode.

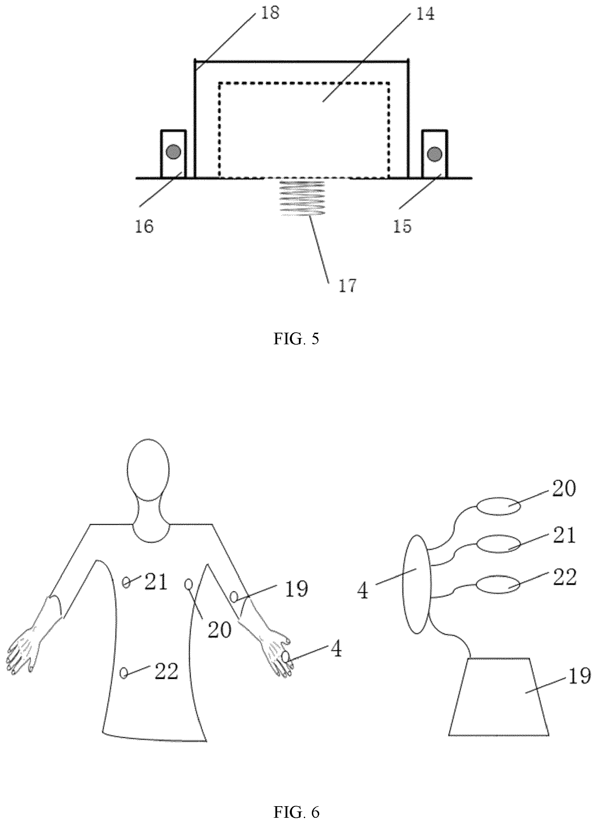

FIG. 4 and FIG. 5 show a top view and a front view of an internal photoplethysmography module 7 according to an embodiment of the present invention. The internal photoplethysmography module 7 is a functional whole, and includes a photodiode 14 protrudingly disposed on the inner circumferential surface 12 of the ring and at least one light emitting diode array that is central symmetrical with respect to the photodiode (FIG. 4 and FIG. 5 show that the internal photoplethysmography module 7 includes two light emitting diode arrays, and each light emitting diode array consists of light emitting diodes 15 and 16), and an elastic device 17 is disposed at the bottom of the photodiode 14. When the ring is worn, the elastic device 17 is pressed, so that the photodiode 14 and the at least one light emitting diode array are in close contact with the finger, and light emitted by the light emitting diode array is attenuated by the finger and then received by the photodiode 14. The light emitting diode array may be formed by light emitting diodes with different wavelengths, for example, red LEDs, infrared LEDs, or green LEDs. In addition, a hood 18 is disposed on the outer circumference of the photodiode 14 and the height of the hood 18 is higher than that of the photodiode 14, so that a problem of light leakage can be effectively solved. The central symmetrical structure of the internal photoplethysmography module 7 and the elastic device at the bottom can effectively ensure pulse wave signal quality and improve accuracy of physiological parameter measurement.

To calibrate the pulse wave transmit time or the pulse wave propagation velocity obtained by using the internal sensor module, the expandable multi-physiological parameter monitoring ring is connected to the external sensor module 5 by using the external module interface 4. Preferably, the external module interface 4 reserves connection ends of all sensor interface circuits. Specifically, as shown in FIG. 6, the external sensor module 5 includes an external pulse wave measurement module and an external electrocardiogram monitoring module. The external pulse wave measurement module implements measurement of a photoplethysmographic (PPG) signal, and may include an external photoplethysmography module 19 placed near the main artery. The external electrocardiogram monitoring module implements measurement of an electrocardiogram (ECG) signal, and may include three electrocardiogram monitoring electrodes 20, 21, and 22. Preferably, the external photoplethysmography module 19 is placed near the brachial artery, the radial artery, or the ulnar artery, the electrode 20 is placed at a position corresponding to the first intercostal space of the midline of the left clavicular of the human front chest, the electrode 21 is placed at a position corresponding to the first intercostal space of the midline of the right clavicular of the human front chest, and the electrode 22 is placed at a position horizontally corresponding to the xiphoid of the right rib end. The external electrocardiogram monitoring electrode is placed at the standard lead position, and the external photoplethysmography module is located near the main artery, and fits with the standard model. Therefore, correlation between the pulse wave transit time (or the pulse wave propagation velocity) and arterial blood pressure is good.

When blood pressure monitoring is started, the internal sensor module cooperates with the external sensor module. The microprocessor module simultaneously obtains measurement signals from the internal sensor module and the external sensor module, and processes the measurement signals to obtain a difference between a measurement signal of the internal sensor module and a measurement signal of the external sensor module. Preferably, the microprocessor module processes the measurement signals to obtain a difference between a pulse wave transit time (or a pulse wave propagation velocity) calculated based on the measurement signal of the external sensor module and a pulse wave transit time (or a pulse wave propagation velocity) calculated based on the measurement signal of the internal sensor module. For example, the microprocessor module can process the measurement signals to obtain a set of data of difference value between the external pulse wave transit time (or the pulse wave propagation velocity) and the internal pulse wave transit time (or the pulse wave propagation velocity), and then calculate an average value of the set of data of difference value as the difference between the external pulse wave transit time (or the pulse wave propagation velocity) and the internal pulse wave transit time (or the pulse wave propagation velocity). When the microprocessor module obtains the measurement signal from only the internal sensor module at a subsequent moment, the pulse wave transmit time (or the pulse wave propagation velocity) obtained based on the internal sensor module is calibrated by using the above difference, and a pulse wave transmit time (or a pulse wave propagation velocity) near the main artery at this moment is obtained, then an accurate arterial blood pressure is further obtained by using a mathematical model between the pulse wave transmit time (or the pulse wave propagation velocity) and the arterial blood pressure. It should be noted that when the blood pressure monitoring is started, the arterial blood pressure value obtained based on the external sensor module can be used as a blood pressure monitoring value, and then connection between the external module interface and the external sensor module can be removed, and even the external module interface can be removed. In this case, the microprocessor module obtains the measurement signal from only the internal sensor module, and the arterial blood pressure value obtained through calculation by using the calibrated pulse wave transit time (or pulse wave propagation velocity) is used as the blood pressure monitoring value.

The embodiments of the present invention describe the structure and function modules of the expandable multi-physiological parameter monitoring ring based on the blood pressure monitoring of the pulse wave transit time method. However, in addition to the blood pressure monitoring, the expandable multi-physiological parameter monitoring ring can have other functions. For example, the internal photoplethysmography module and the internal photoplethysmography analog front end can also implement blood oxygen saturation and pulse rate monitoring. The surface electrode interface and the internal electrocardiogram monitoring analog front end can implement electrocardiogram monitoring. In addition, as shown in FIG. 2, the expandable multi-physiological parameter monitoring ring may include a temperature monitoring module 23 to implement body temperature monitoring, an acceleration module 24 to implement a step counting function, a display module 25 to implement data display, and a rechargeable internal power supply 26 to implement power supply. Gesture control can be implemented according to a data analysis, and data can be transmitted to a remote terminal 27 in a wireless manner. And a vibration motor 28 and the display module 25 can be controlled according to set threshold to implement a data over-limit alarm prompt.

It should be noted that, in the claims and the description of the present patent, relational terms "first", "second", and the like are only used to distinguish one entity or operation from another entity or operation, but are not intended to indicate any actual relationship or order between entities or operations. Moreover, the terms "include", "have", and any other variants thereof are intended to cover non-exclusive inclusion, for example, a process, a method, article, or a device that includes a series of elements not only includes those elements, but includes other elements that are not clearly listed or are inherent to the process, the method, the article, or the device. Without more restrictions, an element that is defined by the phrase "including a" does not exclude the presence of another same element in the process, method, article, or device that includes the element.

Although the present invention has been illustrated and described with reference to the preferred embodiments of the present invention, it should be understood by persons of ordinary skills in the art that various changes in form and details may be made without departing from the spirit and scope of the present invention.

## Claims

1. An expandable multi-physiological parameter monitoring ring, comprising:
a microprocessor module and an internal sensor module connected to the microprocessor module, wherein the expandable multi-physiological parameter monitoring ring further comprising an external module interface for connecting an external sensor module, the external module interface is detachably connected to the microprocessor module;
the microprocessor module is configured to: when the external module interface is connected to the external sensor module, obtain measurement signals from the external sensor module and the internal sensor module, and process the measurement signals to obtain a difference between a measurement signal of the external sensor module and a measurement signal of the internal sensor module; and
when the external module interface is not connected to the external sensor module, calibrate, according to the difference, a physiological parameter obtained by the internal sensor module.

2. The expandable multi-physiological parameter monitoring ring according to claim 1, wherein the physiological parameter is a pulse wave transit time or a pulse wave propagation velocity.

3. The expandable multi-physiological parameter monitoring ring according to claim 1 or 2, wherein the microprocessor module is configured to obtain a difference value between the physiological parameter calculated based on the measurement signal of the external sensor module and the physiological parameter calculated based on the internal sensor module as the difference.

4. The expandable multi-physiological parameter monitoring ring according to claim 1 to 3, wherein the internal sensor module comprises an internal pulse wave measurement module and an internal electrocardiogram monitoring module.

5. The expandable multi-physiological parameter monitoring ring according to claim 1 to 4, wherein the internal pulse wave measurement module comprises an internal photoplethysmography analog front end and an internal photoplethysmography module, and when the ring is worn, the internal photoplethysmography module is in contact with a finger, and the internal photoplethysmography analog front end processes a signal from the internal photoplethysmography module.

6. The expandable multi-physiological parameter monitoring ring according to claim 5, wherein the internal photoplethysmography module comprises a photodiode protrudingly disposed on an inner circumferential surface of the ring and at least one light emitting diode array that is central symmetrical with respect to the photodiode; an elastic device is disposed at the bottom of the photodiode; and when the ring is worn, the elastic device is pressed, so that the photodiode and the at least one light emitting diode array are in close contact with the finger, and light emitted by the light emitting diode array is attenuated by the finger and then received by the photodiode.

7. The expandable multi-physiological parameter monitoring ring according to claim 4, wherein the internal electrocardiogram monitoring module comprises an internal electrocardiogram monitoring analog front end and at least two surface electrode interfaces, the surface electrode interface is used for mounting an electrode, and when the finger ring is worn, the electrode is in contact with a finger, and the internal electrocardiogram monitoring analog front end processes a signal from the surface electrode interface.

8. The expandable multi-physiological parameter monitoring ring according to any of the above claims, wherein the ring further comprises a function expansion module connected to the microprocessor module, the internal sensor module and the external module interface are connected to the microprocessor module by the function expansion module; and the function expansion module is configured to receive a control signal of the microprocessor module, so as to enable one or more of the external module interface and respective submodules of the internal sensor module to work.
